Europäisches Patentamt

European Patent Office

Office européen des brevets

(19)

(11) EP 1 348 426 A1

(12) EUROPEAN PATENT APPLICATION

(43) Date of publication:
01.10.2003 Bulletin 2003/40

(51) Int Cl.⁷: **A61K 7/48**, A61L 15/58,
A61L 24/04

(21) Application number: 03447064.1

(22) Date of filing: 27.03.2003

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: 29.03.2002 EP 02447052

(71) Applicant: THE PROCTER & GAMBLE COMPANY
Cincinnati, Ohio 45202 (US)

(72) Inventors:
• Cinelli, Fabio
65125 Pescara (IT)

• Coles, Peter
65830 Kriftel (DE)
• Goldman, Stephen Allen
65013 Citta Sant'Angelo (Pescara) (IT)
• Romano, Mario
65125 Pescara (IT)

(74) Representative: Canonici, Jean-Jacques et al
NV Procter & Gamble Services Company SA,
Temselaan 100
1853 Strombeek-Bever (BE)

(54) **Hydrogel adhesives for use on hair or fibre-populated surfaces**

(57) The present invention relates to the use, for adhesion on hair or fiber-populated surfaces, of hydrogel adhesive characterized by a G'$_{25}$, (1 rad/sec) below 1000 Pa, and containing weak acid monomer units at selected degree of neutralization, not only resulting in embedding of the hair or fiber and good adhesion properties on the surface, but also in excellent results in terms of cohesiveness, and peel force.

The invention also encompasses Personal Care products and Surface Care Articles containing the hydrogel compositions herein.

EP 1 348 426 A1

Printed by Jouve, 75001 PARIS (FR)

**Description**

Field of the Invention

The present invention relates to hydrogel adhesives for attachment to hair or fibers populated surfaces in particular skin, and to Personal Care or Surface Care products containing such hydrogels.

Background of the Invention

While the use of hydrogel body adhesives in the of medical field, such as skin electrodes, transdermal drug delivery and wound healing, has been known for several years, hydrogel body adhesives have been disclosed more recently for use in consumer products such as absorbent articles and human waste-management articles; representative of the latter disclosures are EP 1 025 823 and EP 1 025 866, where certain needs specific for such consumer products waste-management products, are addressed, including secure attachment, painless removal and stability of adhesion in presence of excess moisture.

However a particular problem which has not been addressed in said prior art is the ability of the adhesive to adhere directly to a skin surface which has hair present, particularly areas of dense hair growth such as occurs in, e. g., the genital area. Typically the prior art teaches especially in wound care applications, and for incontinence applications such as ostomy articles to remove the hair follicles present on the skin by shaving for example prior to the application of the adhesive. However, such practices whilst moderately acceptable in medical circumstances are not desirable for products which are intended for everyday use for example absorbent articles such as sanitary napkins or incontinence devices such as human waste management devices. The absence of the hair on the skin thereby allows a gasket to be formed with the skin which is necessary in order to ensure the containment of the excreted fluids within the particular device of usage. The prior art does not however address how such gasketing can be achieved without the indignity of removing hair.

Not only does the presence of hair on the skin increase the difficulty to obtain optimum adherence to the skin of the wearer, in addition due to the adhesion which occurs between the hair and the adhesive itself, which typically involves some embedding of the hair within the hydrogel adhesive gel matrix, the removal of the adhesive from the wearer is also extremely painful and is significantly increased versus a skin surface where any hair present has been previously removed.

Thus is it highly desirable to provide a hydrogel adhesive for adhesion to the skin which adheres on skin surfaces which have hair present and which have a reduced pain level on removal of the adhesive. It is further desirable to provide an adhesive which adheres to the skin of a hair populated skin surface but which only has minimal and preferably substantially no adhesion to the hair itself.

It is further desirable to provide an adhesive which can embedd the hair and adhere directly to the skin, thus providing good gasketing, while still allowing painless removal.

It is still another objective of the present invention to provide an adhesive that exhibits an ability to adhere to skin upon reapplication, particularly multiple reapplication for example when the adhesive is misplaced, whilst still allowing painless removal.

It has now been surprisingly found that the above drawbacks will be substantially alleviated by the use of hydrogel adhesive with rheology characteristics as defined hereinafter; through said use, attachment to the skin is secure, even if hair is present, the hydrogel is pleasing to the skin upon application, and yet causes no discomfort upon removal and minimal residues.

The adhesion on moist or wet skin and the cohesiveness have been found to be particularly enhanced for the selected hydrogel adhesives of the present invention.

It has also been found that the benefits of the use and of the Hydrogels herein can be applied not only to skin populated with hair, but to more generally to surfaces populated with fibers, and thus the present invention also applies to the use of hydrogel with selected characteristics, for surface care articles.

Summary of the Invention

In a first embodiment herein, the present invention is directed to hydrogel adhesives, capable of adhering on hair or fiber-populated surfaces, comprising 10-60 wt% of a cross-linked hydrophilic polymer; 5-80 wt% of a water-soluble nonionic humectant preferably glycerol, and 10-85 wt% water, said adhesive having an elastic modulus at a temperature of 25ºC, $G'_{25}$ selected such that $G'_{25}$ (1 rad/sec) is less than 1000Pa preferably from 100 to 700 Pa, and wherein said hydrophilic polymer comprises at least 10 mole%, preferably at least 30% mole, more preferably at least 50% mole, most preferably at least 80% mole, of a weak-acid monomer preferably acrylic acid, said weak-acid monomer being at least 50 mole%, preferably at least 70mole%, most preferably at least 90 mole % in its acid form.

The hydrogel adhesives herein preferably have the ratio of $G''_{25}$ (1rad/sec)/$G'_{25}$ (1rad/sec) is in the range of 0.15 to 0.65 preferably 0.15 to 0.35 and the peel strength force on dry skin is in the range than 0.3 to 3N/cm preferably 0.3 to 2N/cm.

In a second and a third embodiment herein, the present invention provides Personal Care Products capable of adhering to hair-populated skin, and Surface Care Articles capable of adhering to fiber-populated surface, said Personal Care Products and said Surface Care Articles comprising a hydrogel adhesive as defined in the first embodiment.

Detailed Description

In a first embodiment herein, the present invention is directed to hydrogel adhesives, capable of adhering on hair or fiber-populated surfaces, comprising 10-60 wt% of a cross-linked hydrophilic polymer; 5-80 wt% of a water-soluble nonionic humectant preferably glycerol, and 10-85 wt% water, having an elastic modulus said adhesive having a viscous modulus at a temperature of 25oC, $G'_{25}$ selected such that $G'_{25}$ (1 rad/sec) is less than 1000Pa preferably from 100 to 700 Pa, and wherein said hydrophilic polymer comprises at least 10 mole%, preferably at least 30% mole, more preferably at least 50% mole, most preferably at least 80% mole, of a weak-acid monomer preferably acrylic acid, said weak-acid monomer being at least 50 mole%, preferably at least 70% mole, most preferably at least 90 mole % in its acid form.

The polymerization of the monomers preferably takes place in presence of the nonionic humectant and water and cross-linking creates a 3-dimensional matrix for the polymer, also referred to as gel form and hydrogel, as will be described in more detail hereinafter.

A critical factor of the hydrogels herein is rheology, is particularly the elastic behavior.

Rheology

The viscous behavior of an adhesive can be interpreted to represent an indication of the ability of the adhesive to quickly attach and securely adhere to a particular surface. The elastic behavior can be interpreted as an indication of the "hardness" behavior of the adhesive. Its value is also important for good initial attachment. Their combination is believed to be an indicator of the required force upon removal. The relation between elastic and viscous modulus is considered to be an indication on which fraction of the removal energy will be dissipated within the adhesive and which fraction is available to trigger the actual removal.

While not being bound by theory, it is believed that for hydrogels applied to skin, the rheological properties at T=37°C are most relevant to adhesion and removal properties. However, for the hydrogels of this invention, it has been found that the rheology properties are only at most moderately sensitive to temperature in the range of 25-37°C. Thus, for the purpose of this invention, it is convenient to specify the rheological properties at a temperature of 25°C. The adhesive has an elastic modulus (also referred to as storage modulus) at a temperature of 25°C abbreviated $G'_{25}$, a viscous modulus at a temperature of 25°C of $G''_{25}$, and the ratio of $G''_{25}$ / $G'_{25}$ at 25°C, referred to as tan $\delta_{25}$.

The elastic modulus (G') of the adhesive is a significant factor which influences the ability of hairs or fibers to be embedded within the hydrogel. Hydrogels with high values of G' are typically too stiff for hairs to be embedded to a significant degree and, for those hairs that do become embedded, too rigid to allow for facile removal without pain. The $G'_{25}$ (1 rad/sec) of the present invention is selected to be less than 1000 Pa, preferably in the range of from 100Pa to 1000Pa, more preferably in the range of 100-700 Pa, and even most preferably in the range of 100-500 Pa.

The nature of the hair or fibers also affects the ability of the hair or fiber to penetrate into the hydrogel adhesive. For hair, it has been found that, in particular, the caliper, length, density, and curliness of the hair, which can vary with hair type, impacts the upper value of G' consistent with good penetration of hair into the hydrogel, allowing good gasketing and adhesion of the hydrogel to the underlying skin. It has been determined that the following equation relates the upper value of $G'_{25}$, (1 rad/s) in Pascals (Pa) consistent with good hair penetration to hair characteristics, based on hair from a referenced 1sqcm area.

$$G'_{25} \text{ (1 rad/sec)} < E/(W*C)$$

wherein :

W = weight of hair per unit area of skin in $g/cm^2$
C = hair curliness factor (ratio of length of stretched to unstretched hair)
E = Numerical Factor for Hair Penetration in units of $Pa*g/cm^2$

We have found that for essentially complete penetration of hair the value for E is 24, preferably 19, even more preferably 9. Thus, for example, if the average value of W for a population type in a relevant 1sqcm area for adhesion is determined to be 0.02 g/cm$^2$, the average curliness factor for this hair based on microscopy analysis of the removed hairs is 1.2, then for E = 24, the value of $G'_{25}$, must be less than 1000 Pa to ensure good embedding of the hair into the hydrogel and thus good gasketing.

The hair or fiber-embedding performance of the adhesive hydrogels herein can be measured by the degree of gasketing provided to the surface to which the hydrogels are applied; and a test method is described hereinafter. The hydrogels herein provide a degree of gasketing significantly higher compared to adhesive hydrogels having a elastic modulus above the present claimed range.

As mentioned above, the viscous modulus of the adhesive is also a significant factor which influence performance, and thus the $G''_{25}$ (1 rad/sec) of the present invention lie in the range of from 50 Pa to 1000 Pa, preferably from 100Pa to 700Pa.

Furthermore, the value of tan $\delta_{25}$ (1rad/sec) directly influences the cohesiveness of the adhesive hydrogels, thus the ability of the hydrogel to disengage from the surface, the hair of the fibers, without leaving residues. The tan $\delta_{25}$ (1rad/sec) for the hydrogel herein should preferably be less than 0.65, preferably in the range from 0.15 to 0.55, more preferably in the range of 0.15-0.35.

Peel Force

It is a preferred characteristic of the hydrogels herein that their peel force, despite the fact the elastic modulus and tan $\delta$ have relatively low values (to ensure respectively good hair or fiber embedding and a high level of cohesiveness), is maintained at an appropriate, value allowing to exhibit excellent adhesion performance on surfaces such as skin. In order to ensure the required skin adhesion initially, and preferably over the entire period of wearer, the adhesive has a peel strength on dry skin of from 0.1 N/cm to 5N/cm, preferably from 0.3 N/cm to 3N/cm most preferably 0.3 N/cm to 2 N/cm as determined according to the test method described herein. The peel force of the hydrogel herein, as measured on dry skin, should be in the range of from 0.1 to 5 N/cm, preferably 0.3 to 3N/cm.

Main Ingredients

According to the present invention the 3-dimensional matrix also referred to herein as a gel, comprises as an essential component a polymer which can be physically or chemically cross-linked. The polymer may be naturally or synthetically derived. The uncrosslinked polymer includes repeating units derived from vinyl alcohols, vinyl ethers and their copolymers, carboxy vinyl monomer, vinyl ester monomers, esters of carboxy vinyl monomers, vinyl amide monomers, hydroxy vinyl monomers, cationic vinyl monomers containing amines or quaternary groups, N-vinyl lactam monomer, polyethylene oxides, polyvinylpyrrolidon (PVP), acrylics such as hydroxyethylmethacrylate, methoxydiethoxyethyl methacrylate, hydroxydiethoxyethyl methacrylate, acrylic acid and acrylates and sulphonated polymers such as acrylamide sulphonated polymers, sulphopropylacrylates and mixtures thereof. Alternatively, the uncrosslinked polymer may be a homopolymer or copolymer of a polyvinyl ether, or a copolymer derived from half ester of maleic ester. Similarly any other compatible polymer monomer units may be used as copolymers such as for example polyvinyl alcohol and polyacrylic acid or ethylene and vinyl acetate.

As another alternative, the polymers may be block copolymer thermoplastic elastomers such as ABA block copolymers such as styrene-olefin-styrene block copolymers or ethylenepropylene block copolymers. More preferably such polymers include hydrogenated grade Styrol/Ethylene-Butylene/Styrol (SEBS), Styrene/Isoprene/Styrene (SIS), and Styrol/Ethylene-Propylene/Styrol (SEPS).

Particularly preferred polymers are acrylics, sulphonated polymers such as acrylamide sulphonated polymers, vinyl alcohols, vinyl pyrrolidine, polyethylene oxide and mixtures thereof.

The polymers herein can also be made of monomers which are selected from strong-acid monomers, weak-acid monomers, nonionic, cationic or zwitterionic monomers.

Strong-acid monomers is defined in relation to their pKa, which must be below 3. The pKa is measured by titration of the acid with strong base in aqueous solution according to methods well known in the art. The said strong-acid monomers are preferably selected from the group of olefically unsaturated aliphatic or aromatic sulfonic acids such as 2-acrylamido-2-methylpropanesulfonic acid, 3-sulphopropyl (meth)acrylate, 2-sulfoethyl (meth)acrylate, vinylsulfonic acid, styrene sulfonic acid, allyl sulfonic acid, vinyl toluene sulfonic acid, methacrylic sulfonic acid and the like. Particularly referred strong-acid monomers are 2-acrylamido-2-methylpropanesulfonic acid, 3-sulfopropyl (meth)acrylate, 2-sulfoethyl (meth)acrylate.

Weak acid monomers are defined in relation to their pKa, which must be above 3. The said monomers are preferably selected from the group of olefinically unsaturated carboxylic acids and carboxylic acid anhydrides such as acrylic acid, methacyclic acid, maleic acid, itaconic acid, crotonic acid, ethacrylic acid, citroconic acid, fumaric acid, $\delta$-

sterylacrylic acid and the like. Particularly preferred weak-acid monomers are acrylic acid and methacrylic acid.

Examples of nonionic monomers include N,N-dimethylacrylamide, acrylamide, N-isopropyl acrylamide, hydroxyethyl (meth)acrylate, hydroxypropyl (meth)acrylate, alkyl (meth)acrylates, N-vinyl pyrrolidone and the like. Examples of cationic monomers include N,N-dimethylaminoethyl (meth)acrylate, N,N-dimethylaminoethyl (meth)acrylamide and the respective quaternary salts and the like.

Selected level of ionization of certain monomers

In a first embodiment herein, the present invention is directed to hydrogel adhesive compositions capable of adhering to hair or fiber-populated surfaces, which exhibit particular good performance in terms of cohesiveness and peel force, as well as adhesion properties on wet or moist skin.

In order to provide optimal cohesiveness/peel strength performance, in accordance with the values disclosed above, it has been found that the hydrophilic polymers of the preferred hydrogels herein, should comprise at least 10 mole% of weak-acid monomer units as described above, said weak-acid monomer being at least 50 mole% in its acid form. Preferably, such polymers should contain at least 30 mole% of weak-acid monomer units, most preferably at least 50 mole%, even most preferably at least 80% mole.

Said weak-acid monomers should preferably be present in at least 70 mole% in their acid form, preferably at least 90 mole% in their acid form.

The weak-acid monomer units herein should preferably be selected from acrylic acid and methacrylic acid, most preferably acrylic acid.

In order to provide excellent adhesion performance on wet or moist skin, the level of monomers units in salt form in the polymers making the preferred hydrogels herein, should be kept below 30 mole% of all monomer units, preferably below 20 mole%.

A most preferred hydrogel composition according to the present invention contains 10-60 % of a cross-linked hydrophobic polymer contains at least 90 mole %, preferably 95 mole% weak acid monomer units, being from 85 mole% to 95 mole% in its acid form, the preferred weak acid being acrylic acid.

It has been found that the level of monomer units in salt form directly impacts the rate at which the hydrogel absorbs physiological fluid such as urine, and thus the hydrogels of the present invention have been found to exhibit a saline absorption rate of less than $2.5 \times 10^{-3}$ g/cm$^2$ /sec$^{0.5}$ preferably less than $2.0 \times 10^{-3}$ g/cm$^2$, even more preferably less that $1.5 \times 10^{-3}$ g/cm$^2$ /sec$^{0.5}$ according to the test method described hereinafter. Without being bound by theory, it is believed that the disassociation of the counterions of monomer units in salt form from the polymer decreases the osmotic driving force for hydrogel swelling and thus the driving force for absorption of physiological fluids such as urine. This decreases the rate for absorption of these fluids. By reducing the rate of absorption for fluids in contact with the hydrogel, the quantity of absorbed fluid is decreased, thus reducing the degree to which exposure to physiological fluids impacts adhesion and cohesion properties.

Saline absorption rate is measured by exposing the adhesive surface of a circular section of the hydrogel to excess saline solution under conditions where (i) the hydrogel is restrained from swelling in the lateral directions and (ii) it is allowed to swell in the z-direction (perpendicular to the plane of the hydrogel) under a confining pressure of 0.3 psi (2.07 kPa) by absorption of 0.9% saline solution. The quantity of saline solution absorbed as a function of time is measured and the saline absorption rate is calculated as described in detail below from the variation of absorbed saline versus time.

The selection of weak-acid monomers as the predominant monomer units for the hydrogel of the present invention, is driven by the following considerations:

Without being bound by theory, it is believed that decreased hydrophilicity of the hydrogel surface improves the adhesion of the hydrogel, especially to hydrophobic surfaces such as skin. It is believed that the ability of the hydrogel to spread onto and into the interstices of the skin surface is aided when the surface energy of the hydrogel is closer to the surface energy of the skin. It is believed that decreased surface hydrophilicity of the hydrogel is particularly important for less-deformable and less-flowable hydrogels having relatively higher values of storage modulus and relatively lower values of tan δ (as described below), rheological properties that are desirable for improving the cohesiveness of the hydrogel and reducing the residue left behind on the surface (e.g., skin) when the hydrogel adhesive is repositioned and/or detached after use.

It is also believed that an increased hydrophobicity of the hydrogel surface reduces the rate of absorption of water when the hydrogel is exposed to high humidity environments and increases the capability of the hydrogel to retain its adhesion properties when it does absorb some water in a high humidity environment and/or when the hydrogel comes into direct contact with physiological fluids. It is further believed that the initial contact angle of a droplet of water on the surface of the hydrogel provides a measure of the hydrophilicity of the hydrogel surface, with a higher contact angle indicating a less hydrophilic and more hydrophobic surface.

Without being bound by theory, it is believed that, for the purpose of increasing the contact angle and reducing the rate of saline absorption, the weak-acid monomers of the present invention are particularly useful; when partially neutralized; these weak-acid monomers may contribute to a high contact angle by their ability to transfer protons from acid-form weak-acid monomers within the bulk of the hydrogel to salt-form weak-acid monomers on the surface, thus decreasing the hydrophilicity of the surface. In their acid form, these weak acid monomers also do not contribute significantly to the driving force for absorption of physiological fluids as discussed hereinabove. This decrease in surface hydrophilicity and saline absorption rate is achieved without the need to excessively decrease the bulk hydrophilicity of the hydrogel (e.g. by incorporating significant concentrations of non-ionic monomers.) Such a decrease in bulk hydrophilicity can be detrimental to other desirable properties of the hydrogel, such as its compatibility to skin, its ability to maintain a favorable pH, its ability to absorb sufficient water for adhering to wet skin, its ability to absorb perspiration, etc.

Contact angle is measured by depositing a droplet of distilled water on the surface of the hydrogel and measuring the initial angle of the drop with respect to the surface using the optical method and calculational methods described hereinafter in test methods. In determining the contact angle of the hydrogel, it is important that the measurement not be influenced by the deposition of a surface layer of hydrophobic material on the hydrogel. Such a surface layer could, for example, be transferred from a siliconized release paper applied to the exposed surface of the hydrogel.

The preferred hydrogels herein have a contact angle of at least 40 degrees, more preferably at least 50 degrees, even more preferably at least 60 degrees, even more preferably at least 70 degrees and most preferably at least 90 degrees.

Humectant

The 3-dimensional adhesive matrix also comprises a humectant or mixture of humectants (also referred herein as a plastisizer), which is preferably a liquid at room temperature. The humectant is selected such that the monomer and polymer may be solubilized or dispersed within. For embodiments wherein irradiation cross linking is to be carried out, the humectant is desiderably irradiation cross linking compatible such that is does not significantly inhibit the irradiation cross linking process of the polymer. The components of the humectant mixture are preferably hydrophilic and miscible with water.

Suitable humectants include alcohols, polyhydric alcohols such as glycerol and sorbitol, and glycols and ether glycol such as mono- or diethers of polyalkylene glycol, mono- or diester polyalkylene glycols, polyethylene glycols (typically up to a molecular weight of about 600), glycolates, glycerol, sorbitan esters, esters of citric and tartaric acid, imidazoline derived amphoteric surfactants, lactams, amides, polyamides, quaternary ammonium compounds, esters such as phthalates, adipates, stearates, palmitates, sebacates, or myristates, glycerol esters, including mono/di/tri-glycerides, and combinations thereof. Particularly preferred are polyhydric alcohols, polyethylene glycol (with a molecular weight up to about 600), glycerol, sorbitol and mixtures thereof. Glycerol is especially preferred. The humectant comprises 5-80 wt% of the hydrogel.

An important function of the humectant is to reduce the water activity of the hydrogel to 0.35-0.95, preferably 0.4-0.85, most preferably from 0.45-0.75. Water activity is determined by measuring the equilibrium relative humidity above the hydrogel according to the method described hereinafter in the test methods section.

Suitable humectants include alcohols, polyhydric alcohols such as glycerol and sorbitol, and glycols and ether glycol such as mono- or diethers of polyalkylene glycol, mono- or diester polyalkylene glycols, polyethylene glycols (typically up to a molecular weight of about 600), glycolates, glycerol, sorbitan esters, esters of citric and tartaric acid, imidazoline derived amphoteric surfactants, lactams, amides, polyamides, quaternary ammonium compounds, esters such as phthalates, adipates, stearates, palmitates, sebacates, or myristates, glycerol esters, including mono/di/tri-glycerides, and combinations thereof. Particularly preferred are polyhydric alcohols, polyethylene glycol (with a molecular weight up to about 600), glycerol, sorbitol and mixtures thereof. Glycerol is especially preferred. The humectant comprises 5-80 wt% of the hydrogel.

An important function of the humectant is to reduce the water activity of the hydrogel to 0.35-0.95, preferably 0.4-0.85, most preferably from 0.45-0.75. Water activity is determined by measuring the equilibrium relative humidity above the hydrogel according to the method described hereinafter in the test methods section.

Polymerization conditions

According to the present invention the polymer component of the adhesive can be physically, chemically or ionically cross linked in order to form the 3 dimensional matrix. Physical cross linking refers to polymers having cross links which are not chemical covalent bonds but are of a physical nature such that for example there are three areas in the 3 dimensional matrix having high crystallinity or areas having a high glass transition temperature or areas having hydrophobic interactions. Chemical cross linking refers to polymers which are linked by chemical bonds. The polymer can be chemically cross linked by radiation techniques such as UV-, E beam-, gamma or micro-wave radiation or by co-polymerizing the monomers with a di/poly-functional crosslinker via the use e.g., of UV, thermal and/or redox polymerization initiators.

Suitable polyfunctional monomer crosslinkers include polyethyleneoxide d(meth)acrylates with varying PEG molecular weights, IRR280 (a PEG diacrylate available from UCB Chemical), trimethylolpropane ethoxylate tri(meth) acrylate with varying ethyleneoxide molecular weights, IRR210 (an alkoxylated tryacrilate: available from UCB Chemicals), trimethyolpropane tri(meth)acrylate, divinylbenzene, pentaerythritol triacrylate, pentaeythritol triallyl ether, triallyl amine, N,N-methylene-bis-acrylamide and other polyfunctional monomer crosslinkers known to the art. Preferred monomer crosslinkers include the polyfunctional diacrylates and triacrylates.

The monomers of the present invention are preferably polymerized via the use of a free radical polymerization initiator. Such free-radical polymerization initiators are well known in the art and can be one or more photoinitiator(s), thermal initiator(s), or redox initiator(s) and be present in quantities up to 5% by weight, preferably from 0.02 % to 2 %, more preferably from 0.02 % to 0.4 %. Photo initiators are preferred. Suitable photo initiators include type I-[]-hydroxyketones and benzyldimethyl-ketals e.g. Irgacure 651 (dimethoxybenzylphenone; available from Ciba Specialty Chemicals) which are believed, on irradiation with UV frequencies, to form benzoyl radicals that initiate polymerization. Particularly preferred photoinitiators include 2-hydroxy-2-methyl-propiophenone (available under the trade name of Darocur 1173 from Ciba Specialty Chemicals), I-hydroxycyclohexylphenylketone (available under the trade name Irgacure 184 from Ciba Specialty Chemicals) and 4-(2-hydroxyethoxy)phenyl-(2-hydroxy-2-methylpropyl) ketone (available under the trade name of Irgacure 2959 from Ciba Specialty Chemicals). Suitable thermal initiators include potassium persulfate and VA044 (available from Wako). Suitable redox initiators include the combination of hydrogen peroxide and ascorbic acid and sodium persulfate and ascorbic acid.

Chemical crosslinking can also be affected after polymerization by use of polyfunctional reagents capable of reacting with polymer functional groups such as ethyleneglycol diglycidyl ether, polyols such as glycerol, and other polyfunctional reagents known to the art.

Crosslinking can also be effected all or in part by ionic crosslinking wherein groups of opposite charge interact via ionic interactions. Suitable ionic crosslinking agents include those known to the art including polyvalent actions such as $Al^{+3}$ and $Ca^{+2}$, d/poly-amines, d/polyquaternary ammonium compounds, including polymeric polyamines and polyquaternary ammonium compounds known to the art.

In preparing adhesive compositions in accordance with the invention, the ingredients will usually be mixed to provide a reaction mixture in the form of an initial pre-gel aqueous based liquid formulation, and this is then converted into a gel by a free radical polymerization reaction as described above. This may be achieved for example using conventional thermal initiators and/or photoinitiators or by ionizing radiation. Photoinitiation is a preferred method and will usually be applied by subjecting the pre-gel reaction mixture containing an appropriate photoinitiation agent to UV light after it has been spread or coated as a layer on siliconised release paper or other solid or porous substrate. The incident UV intensity, at a wavelength in the range from 240 to 420nm is of sufficient intensity and exposure duration (e.g. 10-3000 mW/cm$^2$) to complete the polymerization in a reasonable time. To facilitate the process, it is often preferable to expose the reaction mixture to several UV irradiation sources, in sequence. The processing will generally be carried out in a controlled manner involving a precisely predetermined sequence of mixing and thermal treatment or history.

The total UV irradiation time should preferably be less than 300 seconds, more preferably less than 60 seconds, and even more preferably less than 10 seconds to form a gel with better than 95% conversion of the monomers, preferably more that 99.9% of monomers, even more preferably more than 99.99% of monomers. Those skilled in the art will appreciate that the extent of irradiation will be dependent on the thickness of the reaction mixture, reactivity and concentration of the monomers, concentration of photoinitiator, properties of the humectant, and nature of substrate on to which the reaction mixture is coated and the source of UV.

These timings are for high pressure mercury arc lamps as the source of UV operating at 200 W/cm. The peak intensity of UV reaching the surface of the substrate is approximately 1000m/W/cm$^2$. For a given lamp, the UV intensity is a function of the operating power and distance of the reaction mixture from the UV source. Also, a high-pass UV filter can be employed to minimize exposure to UV intensities of very-low wavelength.

In order to minimize and preferably eliminate the presence of any residual monomers it is important to ensure that the reaction is complete. This is dependent upon a number of factors such as the substrate onto which the adhesive is applied, the type and intensity of the ultra violet light and the number of ultra violet light passes.

Optional Ingredients

Common additives known in the art such as polymerization inhibitors, chain transfer agents, surfactants, soluble or dispersible polymers, buffers, preservatives, antioxidants, pgments, mineral fillers, and the like, and mixtures thereof, may also be comprised within the adhesive composition in quantities up to 10% by weight each respoectively. Preferably, the hydrogels herein should contain no salt or minimum levels, below 1% by weight, preferably below 0.5% by weight.

pH

The pH of the hydrogel composition herein is in the range of from 3 to 6, more preferably 3 to 5.5, most preferably from 3.5 to 5.5, which represents values perfectly compatible with the pH of mammalian skin.
This pH range is directly achievable by the compositions herein, without, without the use of any additional buffering agent, which can have a detrimental impact on the performance and skin friendliness of the hydrogels herein.
The conditions of measure of the pH are described hereinafter in the test methods section.

Personal Care Products

In a second embodiment herein, the present invention is directed to Personal Care Products, capable of adhering to hair-populated skin, which contain a hydrogel adhesive having the characteristics described above, i.e. 10-60% of a cross-linked hydrophilic polymer as described above, 5-80% of a water-soluble nonionic humectant as described above, 10-85% water, and wherein said hydrophilic polymer comprises at least 10 mole%, preferably at least 30% mole, more preferably at least 50% mole, most preferably at least 80% mole, of a weak-acid monomer preferably acrylic acid, said weak-acid monomer being at least 50 mole%, preferably at least 70mole%, most preferably at least 90 mole % in its acid form, said adhesive having a $G'_{25}$ (1 rad/sec) of less than 1000Pa, preferably 100 to 700 Pa, and which preferably has a ratio $G''_{25}$ (1 rad/sec)/ $G'_{25}$ (1 rad/sec) of from 0.15 to 0.65, preferably 0.15 to 0.55, and most preferably 0.15 to 0.35 and a peel strength force on dry skin in the range of from 0.3 to 3N/cm.
For the purpose of the present invention, personal care products means products, disposable or reusable, which are designed to be worn by a human in contact or close proximity to the body in order to achieve a function directed to the person's heath, well-being, comfort or pleasure, and thus require temporary adhesion to the body.
A first type of such articles includes disposable, human waste management devices such as urine, menstrual and faecal management devices.

Disposable Waste-Management Devices

Urine, menstrual or faecal management devices herein include bags having an aperture and a flange surrounding the aperture for adhesive attachment to the uro genital area and or the perianal area of a wearer. Any faecal, menstrual or urine management device known in the art can be provided with an adhesive according to the present invention. Such devices are described for example in WO 99/00084 and WO 99/00085.
The urine, menstrual or faecal management devices herein also includes devices designed to be attached to artificial apertures in the body, such as ostomy/ colostomy devices.
The bag as used in such articles is a flexible receptacle for the containment of urine, menstrual and excreted faecal matter.
The bag is designed to safely contain any entrapped material, typically it will be liquid impermeable, yet it may be breathable. The bag is designed of sufficient strength to withstand rupture in use, also when pressure on the bag is exerted in typical wearing conditions, such as sitting.
The bag may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt.
The human waste management device in particular urine/menstrual management devices according to the present invention may also comprise an additional acquisition layer. The acquisition layer is typically secured to the inner surface of bag. However, the acquisition layer may also be secured to the flange, or both the flange and the inner surface of bag. The acquisition layer is preferably positioned such that it separates the genitalia of the wearer from coming into direct contact with the absorbent material. The acquisition layer is fluid pervious allowing urine/menses to readily pass through so that it may be absorbed by absorbent material.
The bag is provided with an aperture whereby excreted matter is received from the body prior to storage within the bag cavity. The aperture is surrounded by a flange and may be provided in any shape or size, such as circular, oblong, heart shaped and may be symmetrical or asymmetrical, preferably the aperture has an oblong configuration either in the longitudinal or in the transversal direction or in both directions, e.g. the contours of the aperture are in the shape of two ellipses with the respective main axes being substantially perpendicular.
The flange comprises a garment facing surface and a wearer facing surface. In a preferred embodiment these are two large, substantially flat surfaces, however, the flange may also comprise projections designed to fit the perineal or coccygeal area of the wearer.

The flange should be made of soft, flexible and malleable material to allow easy placement of the flange to the perianal area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a basis weight of 5 to 250 $g/m^2$, more preferably 50 $g/m^2$. Other thermoplastic foam materials, or other suitable plastics sheet materials having the described properties of such foams (i.e., softness, pliability, stretchability, and contractibility) might also be used.

The adhesive can be applied to the wearer facing surface of the flange by any means known in the art such as slot coating, spiral, or bead application or printing. Typically the adhesive is applied at a basis weight of from 20$g/m^2$ to 2500$g/m^2$, more preferably from 500$g/m^2$ to 2000$g/m^2$ most preferably from 700$g/m^2$ to 1500$g/m^2$ depending on the end use envisioned. For example, for faecal management devices to be used for babies the amount of adhesive may be less than for faecal management devices designed for active adult incontinence sufferers.

Disposable Absorbent Articles

Another type of personal care articles herein include disposable absorbent articles such as diaper, sanitary napkins, pantiliners, tampons, perspiration pads. Absorbent articles are articles containing an absorbent core, and can be made by any of the ways usual in the art. The application of the adhesive to the wearer facing surface, typically the topsheet surface of an absorbent article should not cause major problems to those skilled in the art since it can be provided by any well known techniques commonly used to apply adhesives. Most preferably the adhesive is provided in a pattern of small incremental areas such as dots or similar.

This invention can be used beneficially on disposable absorbent articles which are applied directly to the skin of a user. The article usually exhibits absorbency for bodily fluids, the protection of the user's garments from soiling, is comfortable to the user, and is easy to produce and to package. The disposable absorbent article is described below by reference to a sanitary napkin or catamenial, however diapers, panty liners, adult incontinence articles, tampons or perspiration pads are also included under the term disposable absorbent articles.

Other Personal Care Products

The present invention the adhesive herein may also find application to other personal care products. The adhesives may for example find utility to adhere functional articles which adhere to the skin such as cosmetic or pharmaceutical delivery articles which provide a substance to the skin such as skin treatment substances, cream, lotions, hormones, vitamins, deodorants, drugs; cosmetic or pharmaceutical delivery articles provide a substance to emanate away from the skin such as insecticides, inhalation drugs, perfumes and; functional articles which are not necessarily attached to the skin, but which require a high residence time on the skin such as decorative cosmetics, (lipstick, eye shadow, stage make-up) and cleaning articles (hand cleaners, face masks and hygienic pore cleansers). Such articles are preferably non-absorbent for bodily liquids.

The adhesive may also in addition find application to attach articles to the skin such as protective articles such as genital-, knee- or elbow-protectors or bandages; clothing such as bras, surgical gowns, or parts of garments during fitting at a tailor; nasal plasters; prosthesis such as breast replacements or wigs; cold wraps e.g. to provide pain relief from bruises and to reduce swelling; thermal wraps comprising thermal cells as disclosed for example in WO 97/36968 and WO 97/49361 to provide relief of temporary and chronic pain such as neck wraps as disclosed in for example US 5 728 146, knee wraps exemplified in WO 97/01311, and back wraps as disclosed for example in US 5 741 318; hearing aids; protective face masks (for the reduction or prevention of inhalation of noxious substances); anti-snoring patches, ornamental articles such as jewellery, earrings, guises, tattoos; goggles or other eye wear, tapes, bandages, dressings of general utility, wound healing and wound management devices; and biomedical skin electrodes such as ECG, EMG, EEG, TENS electrosurgery, defibrillation, EMS and electrodes for facial/beauty applications; and fixation products and/or devices intended to affix patient catheters, tubing leadwires cables, etc.

Surface Care Articles

In a third embodiment herein, the present invention is directed to Surface Care Articles.

For the purpose of the present invention, Surface Care Articles means articles, disposable or removable, which are designed to be temporary applied to a surface in order to achieve a function directed to the treatment of said surface, and thus require temporary adhesion to said surface.

The surfaces to which the articles are particularly adapted are fiber-populated surface which includes any durable fabric used in garment upholstery, carpet as well as disposable fabrics such as nonwovens.

The treatment function to be achieved by the present invention articles can range from the permanent or temporary application of textile finishing, the cleaning of garment, upholstery or carpets, to the application on the durable or disposable fabrics, of substances such as perfumes or other actives designed to emanate from the textile.

An example of a surface-care article according to the present invention is a carpet dusting implement, containing a adhesive hydrogel according to the present invention, applied to carpets as a substitute or a complement to vacuum cleaning; the hydrogel may be present as a roll of disposable wipes rolled onto a mop, used as a carpet cleaning implement; other application of such a mop execution can be envisaged, such a dusting / cleaning of upholstery, curtains and garments.

Test Methods

*1. Rheology*

The rheology of hydrogels is measured at 25°C using a RHEOMETRICS SR 5000 oscillatory rheometer or the equivalent. A sample of thickness of approximately 1mm and diameter of 25 mm is placed between two insulated Parallel Plates of 25mm diameter, controlled at a temperature of approximately 25°C using a Peltier system or equivalent. A Dynamic Frequency Sweep is performed on the hydrogel in either stress or strain mode at an applied strain within the linear elastic response of the hydrogel (e.g., up to a strain of about 10%), with measurements at discrete frequency values between 0.1 and 100 rad/sec. Results are quoted as G', G" and tan delta at frequency values of 1.0 and 100 rad/sec. The hydrogel is aged at least 24 hours before measurement. The average of at least three determinations are reported.

*2. Peel Force on Dry Skin*

The peel force to remove hydrogel from dry skin is measured using a suitable tensile tester, for example an Instron Model 6021, equipped with a 10N load cell and an anvil rigid plate such as the Instron accessory model A50L2R-100. Samples are cut into strips of width 25.4mm and length between about 10 and 20 cm. A non-stretchable film of length longer than the hydrogel is applied to the reverse side of the hydrogel sample (e.g., the substrate side) using double sided adhesive. A suitable film is 23μ thick PET, available from Effegidi S.p.A, 43052, Colorno, Italy. For samples with release paper, the release paper is removed prior to applying the hydrogel to the forearm and then rolling it into place using a compression weight roller to prevent air entrapment between hydrogel and skin. The roller is 13cm in diameter, 4.5cm wide and has a mass of 5Kg. It is covered in rubber of 0.5mm thickness. The free end of the backing film is attached to the upper clamp of the tensile tester and the arm is placed below. The sample is peeled from the skin at an angle of 90 degrees and a rate of 1000mm/min. The average peel value obtained during peeling of the whole sample is quoted as the peel value in N/cm. The average of triplicate measurements is reported.

*3. Peel force on PET*

Peel force to remove hydrogel from poly(ethylene teraphthalate) (PET) film is measured using a suitable tensile tester, for example an Instron Model 6021, equipped with a 10N load cell and attachment for a rigid lower plate, e.g. steel, oriented along the direction of cross-head movement. Freshly produced hydrogel is stored in a closed aluminium bag or similar for at least 12 to 24 hours at room temperature before measuring. A defect free sample of at least 10cm in length is cut from the hydrogel sample. A piece of double sided adhesive, for example type 1524 from 3M Italia S.p.A, I-20090 Segrate, Italy, at least 130mm long and 25.4mm wide is stuck to the back side of the hydrogel laminate. The hydrogel is cut along the tape's outer edges. The second liner is removed from the tape and it is stuck on the rigid base plate. A strip of standard PET of 23μ thickness and no corona treatment, is cut to about 300mm x 40mm. Suitable material would include "Cavilen-Forex" from Effegidi S.p.A, Via Provinciale per Sacca 55, I-43052 Colorno, Italy. The release liner is removed from the hydrogel and the bottom end fixed to the rigid plate by regular tape. The standard substrate is then applied onto the body adhesive using a hand roller once forward and once backward at a speed of 1000 to 5000 mm/min. The roller is 13cm in diameter, 4.5cm wide and has a mass of 5Kg. It is covered in rubber of 0.5mm thickness. The measurement is preferably performed within 10 minutes of application of the substrate.

The free end of the standard substrate is doubled back at an angle of 180 degrees and the rigid plate is clamped in the lower clamp of the tensile tester. The free end of the standard substrate is fixed in the upper clamp of the tensile tester. The peel test is performed at a speed of 1000mm/min. The initial 20mm of peel is disregarded and the average force over the remaining length is quoted as the peel force in N/cm. The average of triplicate measurements is reported.

*4. Removal Pain Grade Test*

The Removal Pain Grade Test is utilized to evaluate the pain during removal from the skin of a wearer of a sample provided with a layer of a adhesive and previously attached to the wearer's skin. The test specifically evaluates the pain upon removal of each sample as compared to the pain obtained by removing a reference sample constituted by a commercial strong medical plaster.

• Sample preparation.

The test is performed on rectangular samples 60x20 mm made of a polyester film 23 μm thick, such as that sold by Effegidi S.p.A. of Colorno (Parma, Italy), provided on one side with a continuous layer of the topical adhesive having the selected thickness, applied with an Acumeter Model LH-1 extruder. The reference sample is a 60x20 mm sample of a of an adhesive non woven fabric available from Beiersdorf A.G. Hamburg, Germany under the Tradename Fixomull stretch.

• Test method.

A panel of six graders is selected for the test. The test is performed in a climatically controlled laboratory maintained at a temperature of 23 °C and a Relative Humidity of 50%. No special treatment of the wearer's skin is required beyond normal cleaning/washing with water and soap. The skin is then allowed to dry for at least two hours before the test to allow the skin to reach equilibrium with the room conditions. Different adhesives are evaluated in the test in comparison with the reference sample R. Each sample is applied by hand by an operator to the inner part of the grader's forearm, being centred between the wrist and the elbow, with the short side of the sample aligned with the length of the arm. The operator exerts on each sample with the palm of the hand the same pressure that is typically applied to cause a medical plaster to adhere to the skin. Each sample is worn for the prescribed time, and then it is removed from the grader's skin by the operator with a slow and smooth pull.

Four series of one reference sample R and the test samples are each applied, worn and then removed from the wearer's skin; each sample is worn for one minute, with a 5 minute wait between two subsequent samples of the same series, and a 15 minute wait between two different subsequent series. The reference sample R is always applied, worn and removed as the first sample of its respective series. The sequence of application/wear/removal of the test samples in each of the first three series is random, provided that no repetition in each series is allowed, and that no sequence is repeated in the first three series. In the fourth series one of the test samples is tested twice, the reference R always being the first one. Overall each sample has to be tested an equal number of times (24 times).

The graders were asked to evaluate each sample using a pain scale ranging from 0 to 10, where 0 corresponds to no pain and 10 corresponds to the pain upon removal of the reference sample R. The pain values for each sample were obtained as a mean of 24 observations.

The results collected from the test were analysed by a statistical analysis program "Comparison of Population Means - Paired Samples", that showed that the differences between the pain values of the samples are statistically significant.

*5. Fiber Embedding Test*

A sample of hydrogel adhesive on a liquid-impermeable substrate with lateral dimensions greater than 35 mm is used for this test. Also used for this test is a high-loft hydrophobic non-woven with basis weight of approximately 35 gsm (for example, Sandler 4378 Sawabond non-woven or equivalent) which has lateral dimensions equal to or greater than that of the hydrogel. Prior to use, the back side of this non-woven (the non-high-loft side) is covered with (i) a double sided adhesive (for example type 1524 from 3M Italia S.p.A, I-20090 Segrate, Italy) and (ii) a piece of standard PET of 23μ thickness and no corona treatment, (for example "Cavilen-Forex" from Effegidi S.p.A, Via Provinciale per Sacca 55, I-43052 Colorno, Italy). The hydrogel sample is applied co-facially on top of the nonwoven with the adhesive side of the hydrogel in contact with the high-loft side. An approximately 35 mm diameter circular piece of the hydrogel/non-woven sandwich structure is cut from the larger piece using a circular punch or equivalent of inner diameter of approximately 35 mm and weighed to an accuracy of at least 0.001 g ($W_{35}$). A compression weight roller with a diameter of approximately 13cm and a mass of approximately 5Kg, which is covered in rubber of approximately 0.5mm thickness, is used to compress the resultant 35 mm diameter piece of hydrogel/non-woven sandwich structure by rolling the compression weight on top of the piece first forward and then backward. The compressed circular piece of hydrogel/non-woven sandwich is positioned with the non-woven side down in a dry container and covered with a cylindrical stainless steel weight of diameter approximately 50 mm and weight of approximately 240 gm. To this container is then added a sufficient volume of 0.9% saline solution, containing approximately 0.01 wt% of Indigo Carmine Blue Dye, to completely cover the sandwich structure. After an immersion time of approximately 60 minutes, the test sample is removed from the saline and the outer surfaces are blotted dry. An approximately 25 mm diameter piece is cut from the center of the 35 mm diameter test sample using a circular punch or equivalent having an inner diameter of approximately 25 mm and weighed to an accuracy of at least 0.001 g ($W_{25}$). By cutting away the outer 5 mm wide ring of the test sample, hydrogel that is swollen solely by diffusive contact with the saline is removed. This central 25 mm diameter piece is also visually checked for the appearance of blue color. The weight gain of the central 25 mm diameter inner section of the hydrogel in percent as a result of immersion of the larger 35 mm test piece in saline is calculated using the following equation:

$$\text{Weight Gain (\%)} = 100 * \{(W_f - W_i)/W_i\} / F$$

where,

$$F = \{W_{35} - (BW_{nws} * A_{35}) - (BW_{substrate} * A_{35})\}/W_{35}$$

and

$W_i = W_{35} * A_{25}/A_{35}$
$W_f = W_{25}$
$W_{35}$ = Dry weight of 35 mm circular piece of hydrogel/non-woven in grams
$A_{35}$ = Area of 35 mm circular piece of hydrogel/non-woven in $cm^2$
$A_{25}$ = Area of 25 mm circular piece of hydrogel/non-woven in $cm^2$
$W_{25}$ = Weight of 25 mm inner circle of hydrogel/non-woven after Immersion in grams
$BW_{nws}$ = Weight per unit area of non-woven structure in units of $g/cm^2$
$BW_{substrate}$ = Weight per unit area of hydrogel substrate in units of g/cm2

The average of at least triplicate determinations are used to calculate the Percentage Weight Gain.

For good fiber embedding into the hydrogel, there are relatively few capillary paths for the saline solution to wick between the hydrogel and the hydrophobic backing of the non-woven and thus there is a low percentage weight gain (e.g., <5%) for the hydrogel in the 25 mm inner circle of hydrogel. There is also a minimal appearance of blue color in this central section of hydrogel. For poor fiber embedding, there are many capillary pathways and thus there is a greater weight gain (e.g., >5%). There is also a significant appearance of blue color in this central section of hydrogel.

*6. Saline Absorption Rate*

A sample of hydrogel having a basis weight of approximately 1 $kg/m^2$ is used for this measurement. A demand absorbency apparatus (as described in detail in Goldman et. al. US 5,599,335) filled with a 0.9 wt% saline solution is used to measure the kinetics of saline solution absorption by the hydrogel. The fritted funnel of the demand-absorbency apparatus is prepared for measurement by rinsing with saline, draining excess fluid, drying, positioning at a fixed height of ~2 mm above the top surface of the fluid reservoir, and sequentially: (i) positioning the valve connecting the fritted funnel so that it is open to the fluid reservoir, (ii) positioning the valve connecting the fritted funnel so that it is open to a drain tube of approximately -5 cm hydrostatic suction for a time period of ~5 minutes, and (iii) positioning the valve connecting the fritted funnel so that it is isolated from both the reservoir and the drain tube. A piston/cylinder apparatus as described in US 5,599,335 is used to confine the hydrogel during the demand absorbency measurement. The cylinder has an inner diameter of 60 mm and a cylinder bottom permeable to saline but impermeable to hydrogel. A stainless steel weight is positioned on top of the piston such that the combined weight of the piston and weight is equivalent to 0.30 psi. From a larger piece of the hydrogel, a punch (or equivalent) is used to obtain a cylindrical section of hydrogel with diameter between 57-60 mm. This hydrogel is centrally positioned inside the cylinder with adhesive surface facing the cylinder bottom and the piston positioned such that the hydrogel is confined between the cylinder bottom and the piston. A variety of approaches can be used to position the hydrogel in the piston/cylinder apparatus such that there is minimal contact between hydrogel and the walls of the cylinder during insertion including: (i) adhering the hydrogel to the piston before inserting the piston and hydrogel into the cylinder, (ii) resting the substrate surface of a hydrogel sample on the inverted piston and slipping the inverted cylinder over the piston and hydrogel, etc. The piston/cylinder apparatus is then centered on the fritted disk of the fritted funnel, the stainless steel weight is inserted into the piston, the fritted funnel cover is positioned onto the funnel, and the experiment is initiated by positioning the valve connecting the fritted funnel so that it is open to the fluid reservoir. The quantity of saline absorbed by the hydrogel is measured as a function of time for a time period of at least one hour. The weight gain between times of 100 and 3600 seconds is fitted to the function:

$$W(t) = (k_s t^{0.5}) + c$$

where $W(t)$ is the weight gain of the hydrogel (in grams) per unit area of the cylinder (28.27 cm$^2$) in units of g/cm$^2$ and $k_s$ is a rate constant in units of g/cm$^2$/sec$^{0.5}$ that describes the kinetics of saline absorption. Weight gain data at times shorter than 100 seconds is excluded from this fit due to a variety of factors that can bias and/or otherwise impact the initial absorption rate. The average slope for at least two determinations is reported as the saline absorption rate.

## EXAMPLES

Preferred Hydrogel compositions according to the present invention are prepared in Examples 1 and 2, with level of weak acid monomer unit in salt form below 50 mole %, and a composition according to comparative example 3 is also prepared.

Example 1

Approximately 18.23 parts of acrylic acid (BASF) are slowly dissolved, with stirring in approximately 25.08 parts of water. To the cooled solution is slowly added, with stirring and cooling, approximately 2.01 parts of 50% sodium hydroxide (NaOH; Aldrich), which is sufficient to convert approximately 10 mole% of the acrylic acid to sodium acrylate. During this addition the temperature is maintained below 25°C. The mixture is stirred for several minutes to allow any precipitated sodium acrylate to redissolve. After dissolution, approximately 55.66 parts of glycerol (Agrar) are added. The final premix is stirred until a complete solution is accomplished. The resultant solution is covered to shield it from light. The composition of this solution, including the water produced via the neutralization of the acid monomers, is given in Table 1.

Aliquots of the above monomer solution are polymerized to form a hydrogel. Prior to polymerization, approximately 0.189 parts of the polyfunctional crosslinker IRR210 (a polyoxyethylene triacrylate crosslinker from UCB) and 0.228 parts of Darocur 1173 (2-hydroxy-2-methyl-propiophenone; Aldrich) is added to approximately 100 parts of the monomer solution and dispersed and/or dissolved with stirring for at least 15 minutes. One fraction of the monomer solution is spread at a basis weight of approximately 1.0 kilograms per square meter onto siliconized release paper (for example, CO.GE.SIL "Silkraft-70g" (Palazzo)), that has been surface treated by wiping with a very-thin layer of Pluronic 6400 surfactant (BASF) to facilitate spreading of the solution. For handling purposes, the release paper is pre-positioned inside a 8.5 cm diameter polystyrene Petri dish. A second fraction of the monomer solution is coated at a basis weight of approximately 1.0 kilogram per square meter onto a thin, porous non-woven substrate (for example Fiberweb 33; Corolind PE; 33g/sqm). This nonwoven is backed by a PET film (Cavilen - Forex; 23 µm), which is attached to the non-woven by 3M 1524 double-sided adhesive. This non-woven is pre-positioned inside a suitable container, for example a 12cm by 12cm polystyrene Petri-dish. The solution is added dropwise over the surface of the non-woven and then spread by gently tilting the box from side-to-side. An IST Model # M20-1(2)-TR-SLC UV Polymer Reactor, equipped with an IST 200 ozone-free arc lamp (Spectrum Type: CKII-OF) is used to effect polymerization. A high-pass UV filter with a frequency cut-off of approximately 310 nM (UV Filter Borofloat T320 from Bedampfungs-technik) is positioned between the lamp and the sample to filter out low-frequency UV irradiation The monomer-coated substrate is irradiated while passing underneath the lamp on a variable-speed belt positioned approximately 13 cm underneath the lamp. The speed of the belt is set at approximately 5 meter/min. The peak output power of the lamp is measured using an UMD-1 power meter (Eta Plus Electronic) and the output intensity of the lamp is adjusted so that the incident peak UV power on the sample is approximately 1000 milliwatt/cm$^2$. Twelve consecutive passes of the sample underneath the lamp is used to polymerize the monomer solution and convert it into a soft adhesive hydrogel. The above procedure is repeated to obtain several samples of hydrogel on each type of substrate.

The resultant hydrogels are analyzed according to the test methods described above. From samples polymerized on release paper, a 25 mm diameter punch is used to obtain a circular sample of hydrogel for measurement of rheology properties. Rheology measurements are made for at least three samples punched from separately-polymerized hydrogel samples on release paper. The average thickness of the sample is obtained from the plate-to-plate separation used for the rheology measurement. Several 10-12 cm by 2.54cm strips are cut from one or more hydrogel sample formed on the non-woven substrate. These strips are used to measure the peel force of the adhesive from PET and from skin. Test results (average of at least three determinations) are summarized in Table 2.

As can be seen from the results in Table 3, G' (1 rad/s) is less than 1000 Pa and tan □ (1 rad/s) is less than 0.55. Neutralizing the acrylic acid to 10% results in a hydrogel with a very-high peel force for attachment to skin, very-good cohesion, and good embedding of hair.

Example 2

The general procedure described in Example 1 is followed except that approximately 16.53 parts of acrylic acid (BASF) are dissolved in approximately 24.54 parts of water. Approximately 5.47 parts of 50% NaOH is added, which is sufficient to convert approximately 30 mole% of the acrylic acid to sodium acrylate. Approximately 57.17 parts of glycerol are added. The composition of this solution, including the water produced via the neutralization of the acid monomers, is given in Table 1. Prior to polymerization, approximately 0.203 parts of IRR210 and 0.228 parts of Darocur 1173 are added to approximately 100 parts of the monomer solution.

Aliquots of this solution are polymerized, yielding a highly adhesive hydrogel. The resultant hydrogels are analyzed as described in Example 1. The results are summarized in Table 2.

As can be seen from the results in Table 2, G' (1 rad/s) is less than 1000 Pa and tan □ (1 rad/s) is less than 0.55. Neutralizing the acrylic acid to 30% results in a high peel force for attachment to skin, while still providing very-high cohesion and good embedding of hair.

Example 3

The general procedure described in Example 1 is followed except that approximately 15.1 parts of acrylic acid are dissolved in approximately 23.05 parts of water. Approximately 11.64 parts of 50% NaOH is added, which is sufficient to convert approximately 70 mole% of the acrylic acid to sodium acrylate. Approximately 55.98 parts of glycerol are added. The composition of this solution, including the water produced via the neutralization of the acid monomers, is given in Table 1. Prior to polymerization, approximately 0.390 parts of IRR210 and 0.228 parts of Darocur 1173 are added to approximately 100 parts of the monomer solution.

The resultant hydrogels are analyzed as described in Example 1. The results are summarized in Table 2.

As can be seen from the results in Table 2, neutralizing the acrylic acid to 70% results in a low peel force for attachment to skin.

Table 1

| Composition of Monomer Solutions Examples 1-3 | | | | | | | |
|---|---|---|---|---|---|---|---|
| Hydrogel (Ex #) | Acrylic Acid (moles/kg) | Na Acrylate (moles/kg) | Na Acrylate (mole%) | Water (wt%) | Glycerol (wt%) | Daracur 1173 (wt%) | IRR210 (wt%) |
| 1 | 2.27 | 0.25 | 10 | 26.5 | 55.7 | 0.23 | 0.19 |
| 2 | 1.61 | 0.69 | 30 | 28.5 | 57.2 | 0.23 | 0.20 |
| 3 | 1.47 | 0.63 | 70 | 31.5 | 56.0 | 0.23 | 0.39 |

Table 2:

| Test Results for Adhesive Hydrogels Examples 1-3 | | | | | | |
|---|---|---|---|---|---|---|
| Hydrogel (Ex #) | G' (25°C) (Pa; 1 rad/s) | G" (25°C) (Pa; 1 rad/s) | Tan □ (1 rad/s) | Peel Force PET (N/cm) | Peel Force Skin (N/cm) | Hair Embedding |
| 1 | 810 | 200 | 0.25 | 0.47 | 0.97 | Good |
| 2 | 740 | 190 | 0.26 | 0.48 | 0.63 | Good |
| 3 | 850 | 180 | 0.21 | 0.22 | 0.20 | Good |

**Claims**

1. A hydrogel adhesive, capable of adhering on hair or fiber-populated surfaces, comprising 10-60 wt% of a cross-linked hydrophilic polymer; 5-80 wt% of a water-soluble nonionic humectant, and 10-85 wt% water, having an elastic modulus at a temperature of 25ºC, $G'_{25}$ selected such that $G'_{25}$ (1 rad/sec) is less than 1000Pa wherein said hydrophilic polymer comprises at least 10 mole% of a weak-acid monomer having a pKa above 3, said weak-acid monomer being at least 50 mole% in its acid form.

2. A hydrogel adhesive according to Claim 1 wherein $G'_{25}$ (1 rad/sec) is in the range from 100 to 700Pa.

3. A hydrogel adhesive according to Claims 1-2 having a viscous modulus at a temperature of 25°C, $G''_{25}$ selected such that $G''_{25}$ (1 rad/sec) is in the range from 50 to 1000Pa, preferably 100 to 700Pa; the ratio of $G''_{25}$ (1rad/sec) /$G'_{25}$ (1rad/sec) is in the range of 0.15 to 0.65, preferably 0.15 to 0.55, most preferably 0.15 to 0.35 and the peel strength force on dry skin is in the range of 0.3 to 2N/cm.

4. A hydrogel adhesive according to Claims 1-3 wherein the weak-acid monomer is present at least at 70 mole%, preferably at least 90 mole%, in its acid form.

5. A hydrogel adhesive according to Claims 1-4 wherein said hydrophilic polymer comprises at least 30% mole of said weak-acid, preferably at least 50% mole, most preferably 80% mole.

6. A hydrogel adhesive according to Claims 1-5 comprising at least 90 mole%, preferably 95% weak acid monomer units, being from 85% to 95% mole% in acid form.

7. A hydrogel adhesive according to Claims 1-6 wherein the weak-acid monomer is selected from acrylic acid and methacrylic acid.

8. A hydrogel adhesive according to Claims 1-7 wherein the level of ionized monomer in said polymer is below 30% mole.

9. A hydrogel adhesive according to Claims 1-8 wherein said water-soluble nonionic humectant is selected from polyhydric alcohols, and is preferably glycerol.

10. A disposable human waste management article which comprises an hydrogel adhesive according to Claims 1-9 wherein said articles comprises a bag, said bag having an aperture and a flange surrounding said aperture, said flange having a wearer-facing surface comprising said hydrogel adhesive.

11. An absorbent article which comprises an hydrogel adhesive according to Claims 1-9 wherein said article comprises an absorbent core and a wearer-facing surface comprising said hydrogel adhesive.

12. An surface care article which comprises a hydrogel adhesive according to Claims 1-9.

## EUROPEAN SEARCH REPORT

**European Patent Office**

**Application Number**

EP 03 44 7064

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00 07636 A (COLES PETER ;MUNRO HUGH SEMPLE (GB); YASIN MOHAMMED (GB); CINELLI) 17 February 2000 (2000-02-17) * page 4, line 29 - page 5, line 21 * * page 9, line 7 - page 10, line 30 * * page 15, line 2 - page 16, line 18 * * page 22, line 18 - page 23, line 27 * | 1-12 | A61K7/48 A61L15/58 A61L24/04 |
| X | EP 1 026 219 A (FIRST WATER LTD) 9 August 2000 (2000-08-09) * page 3, line 2 - line 54 * * examples 1-8 * * claims 1-16 * | 1-12 | |
| X | US 2002/026005 A1 (MUNRO HUGH SEMPLE) 28 February 2002 (2002-02-28) * page 2, left-hand column, paragraph 2 - right-hand column, paragraph 6 * * page 3, left-hand column, paragraph 1 - paragraph 7 * * page 5, right-hand column, paragraph 4 - page 6, left-hand column, paragraph 3 * * examples 1-13 * | 1-12 | |
| P,X | EP 1 245 240 A (PROCTER & GAMBLE) 2 October 2002 (2002-10-02) * page 2, line 13 - line 36 * * page 3, line 52 - page 4, line 6 * * page 5, line 43 - page 6, line 35 * * examples 1,2 * * claims 1-14 * | 1-12 | **TECHNICAL FIELDS SEARCHED (Int.Cl.7)** A61K A61L A61F C09J |

The present search report has been drawn up for all claims

| Place of search | Date of completion of the search | Examiner |
|---|---|---|
| THE HAGUE | 30 June 2003 | Menidjel, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 03 44 7064

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2003

| Patent document cited in search report | | Publication date | Patent family member(s) | | Publication date |
|---|---|---|---|---|---|
| WO 0007636 | A | 17-02-2000 | AU | 5180199 A | 21-02-2000 |
| | | | AU | 5180799 A | 28-02-2000 |
| | | | AU | 5180999 A | 21-02-2000 |
| | | | AU | 747610 B2 | 16-05-2002 |
| | | | AU | 5181199 A | 28-02-2000 |
| | | | AU | 5181799 A | 28-02-2000 |
| | | | BR | 9913350 A | 02-07-2002 |
| | | | BR | 9913351 A | 16-07-2002 |
| | | | CA | 2338692 A1 | 10-02-2000 |
| | | | CA | 2338715 A1 | 10-02-2000 |
| | | | CA | 2338844 A1 | 17-02-2000 |
| | | | CA | 2339139 A1 | 17-02-2000 |
| | | | CA | 2339203 A1 | 17-02-2000 |
| | | | CN | 1315875 T | 03-10-2001 |
| | | | CN | 1320047 T | 31-10-2001 |
| | | | EP | 1100556 A1 | 23-05-2001 |
| | | | EP | 1100554 A1 | 23-05-2001 |
| | | | EP | 1100557 A1 | 23-05-2001 |
| | | | EP | 1104312 A1 | 06-06-2001 |
| | | | EP | 1100555 A1 | 23-05-2001 |
| | | | WO | 0006214 A1 | 10-02-2000 |
| | | | WO | 0007636 A1 | 17-02-2000 |
| | | | WO | 0006215 A1 | 10-02-2000 |
| | | | WO | 0007637 A1 | 17-02-2000 |
| | | | WO | 0007638 A1 | 17-02-2000 |
| | | | JP | 2002521140 T | 16-07-2002 |
| | | | JP | 2002521526 T | 16-07-2002 |
| | | | JP | 2002522121 T | 23-07-2002 |
| | | | JP | 2002522122 T | 23-07-2002 |
| | | | JP | 2002522563 T | 23-07-2002 |
| | | | US | 2002037270 A1 | 28-03-2002 |
| | | | US | 2002034492 A1 | 21-03-2002 |
| | | | US | 2002035320 A1 | 21-03-2002 |
| EP 1026219 | A | 09-08-2000 | EP | 1026219 A1 | 09-08-2000 |
| | | | AU | 2306000 A | 25-08-2000 |
| | | | CA | 2360642 A1 | 10-08-2000 |
| | | | EP | 1163309 A1 | 19-12-2001 |
| | | | WO | 0046319 A1 | 10-08-2000 |
| | | | JP | 2002536091 T | 29-10-2002 |
| | | | US | 2002015689 A1 | 07-02-2002 |
| US 2002026005 | A1 | 28-02-2002 | AU | 2306100 A | 25-08-2000 |
| | | | CA | 2361871 A1 | 10-08-2000 |
| | | | EP | 1150721 A1 | 07-11-2001 |
| | | | WO | 0045864 A1 | 10-08-2000 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**                     EP 03 44 7064

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2003

| Patent document cited in search report | | Publication date | Patent family member(s) | | Publication date |
|---|---|---|---|---|---|
| US 2002026005 | A1 | | JP | 2002536073 T | 29-10-2002 |
| EP 1245240 | A | 02-10-2002 | EP | 1245240 A1 | 02-10-2002 |
| | | | WO | 02078757 A1 | 10-10-2002 |
| | | | WO | 02078756 A1 | 10-10-2002 |

EPO FORM P0459

For more details about this annex : see Official Journal of the European Patent Office, No. 12/82